# EUROPEAN PATENT SPECIFICATION

(11) **EP 2 744 447 B1**
(45) Date of publication and mention of the grant of the patent: **10.10.2018**
(21) Application number: 12787072.3
(22) Date of filing: 15.08.2012
(51) Int. Cl.: A61F 2/02, B29C 51/00, B29C 53/00, B29C 53/84, B32B 1/00

(54) **THERMOPLASTIC MULTILAYER ARTICLE**
THERMOPLASTISCHE MEHRSCHICHTIGE ARTIKEL
ARTICLE MULTICOUCHE THERMOPLASTIQUE

(30) Priority: 16.08.2011 US 201161524046 P
(43) Date of publication of application: 25.06.2014
(73) Proprietor: DePuy Synthes Products, LLC, Raynham, MA 02767 (US)
(72) Inventor: THORWARTH, Goetz, CH-4436 Oberdorf (CH); VOISARD, Cyril, CH-4436 Oberdorf (CH)
(74) Representative: Klunker IP Patentanwälte PartG mbB
(86) International application number: PCT/IB2012/001964
(87) International publication number: WO 2013/024355

(56) References cited:
- US-A1- 2002 180 101
- US-A1- 2005 021 131
- US-A1- 2008 154 368
- US-B1- 6 221 075

## Description

### FIELD OF THE INVENTION

The present invention generally relates to multilayer articles. More particularly, the present invention relates to multilayer articles suitable for implantation into a patient. Some embodiments of the invention relate to multilayer articles having one or more thermoplastic layers and one or more base layers. Further embodiments of the invention relate to methods for making multilayer articles.

US 2005/0021131 A1 discloses a stent made from one or more polmer layers, wherein the stent may change its shape at different temperatures depening on the glass transition temperatures of the polmer layers. US 6,221,075 B1 discloses bone plates made of biocompatible, bioabsorbable thermoplastic material. US 2002/0180101 A1 discloses an orthopedic implant formed from a sheet of moldable material. US 2008/0154368 A1 discloses an orthopedic implant comprising a biocompatible sheath and a curable material sealed within the sheath

### BRIEF SUMMARY OF THE INVENTION

According to the invention, a multilayer article includes a thermoplastic layer having a first surface and a second surface opposite the first surface and a base layer have a first base surface and a second base surface opposite the first base surface, wherein the first base surface of the base layer is permanently bonded to the second surface of the thermoplastic layer, wherein the thermoplastic layer has a first glass transition temperature, T_{g}^{I}, and wherein the multilayer article is rigid at temperatures less than T_{g}^{I} and is flexible at temperatures greater than T_{g}^{I}. In some embodiments, the first base surface of the base layer is adjacent to the second surface of the thermoplastic layer. In some embodiments, the multilayer article includes a plurality of thermoplastic layers and plurality of base layers arranged in alternating layers. In further embodiments, the multilayer article includes an interlayer, for example, a diamond-like carbon layer, positioned between the thermoplastic layer and the base layer.

In some embodiments, the thermoplastic layer is made from a material independently selected from the group consisting of: polyethylene terephthalate, polycaproamides, polytrimethylene carbonates, polyglycolide-co-trimethylene carbonates, poly(PBA-carbonates), poly(beta-hydroxybutyrate), polyhydroxybutyrate-hydroxyvaleric acids, and combinations thereof. In some embodiments, the thermoplastic layer is made from a material comprising a material selected from the group consisting of: polyethylene terephthalate, polycaproamides, polytrimethylene carbonates, polyglycolide-co-trimethylene carbonates, poly(PBA-carbonates), poly(beta-hydroxybutyrate), polyhydroxybutyrate-hydroxyvaleric acids, and combinations thereof.

According to the invention, the base layer is made from a biocompatible metallic material. In some embodiments, the biocompatible metallic material is independently selected from the group consisting of: titanium, titanium alloys, cobalt-chrome alloys, cobalt-chrome-molybdenum alloys, molybdenum alloys, tantalum, nitinol, GUMMETAL®, stainless steel and spring steel.

In other embodiments, which are not part of the invention, the base layer is made from a second thermoplastic material having a second glass transition temperature, T_{g}^{II}, which is at least 50 °C higher than T_{g}^{I}. In some variations of these embodiments, the second thermoplastic material is independently selected from the group consisting of: polycarbonate, polyaryl ether ketone, polyether ether ketone, polyether ketone ketone, fiber reinforced polyether ether ketone and fiber reinforced polyether ketone ketone, and combinations thereof. In some embodiments, an interface between the molten thermoplastic material and second thermoplastic material forms a contact angle of less than 5°.

In some embodiments, a multilayer article of the present invention has a thickness ranging from 0.1 mm to 3.0 mm. In some embodiments, the multilayer article has a thickness ranging from 0.1 mm to 1.5 mm.

In some embodiments, the present invention includes a bone plate made from a multilayer article. In other embodiments, the present invention includes, a thermally releasing spring element having a coil spring shape made from a multilayer article.

A method to form a multilayer article according to the present invention includes the steps of: (a) providing a base layer have a first base surface and a second base surface opposite the first base surface, the base layer having a thickness ranging from 0.5 mm to 0.01 mm; and (b) applying a thermoplastic layer to the first base surface of the base layer, the thermoplastic layer having a first glass transition temperature, T_{g}^{I}. In some embodiments, the method further includes repeating step (a) to form a multilayer article of at least ten alternating layers of the thermoplastic layer and the base layer. The method includes applying heat and pressure to the layers. In one embodiment, the layers are heated to at least 200 °C for at least 1 minute to permanently affix the thermoplastic layers to the surface of the base layer. According to the invention, the layers are heated to at least 10 °C higher than the melting temperature Tₘ of the thermoplastic for at least 1 minute to permanently affix the thermoplastic layers to the surface of the base layer. The multilayer article is rigid at temperatures less than T_{g}^{I} and is flexible at temperatures greater than T_{g}^{I}.

According to the invention, the base layer is made from a biocompatible metallic material. In other embodiments, which are not part of the present invention, the base layer includes a second thermoplastic material having a second glass transition temperature, T_{g}^{II}, which is at least 50 °C higher than T_{g}^{I}.

In some embodiments, a method according to the present invention further includes coating the surface of the biocompatible metallic material with diamond like carbon to form a diamond like carbon interlayer positioned between the thermoplastic layer and the base layer. In further embodiments, the method also includes activating the diamond like carbon layer with oxygen plasma.

In some embodiments, a method according to the present invention further includes cooling the multilayer article, and shaping the multilayer article to form a deformable implant. In other embodiments, the method includes rolling the multilayer article to form a coil spring shape.

A method for securing a plurality of bone fragments according to one embodiment, the steps of: providing a laminate plate including: alternating layers of a thermoplastic layer and a base layer, the thermoplastic layer having a first surface and a second surface opposite the first surface and the base layer have a first base surface and a second base surface opposite the first base surface, wherein the first base surface of the base layer is adjacent to and permanently bonded to the second surface of the thermoplastic layer, wherein the thermoplastic layer has a first glass transition temperature, T_{g}^{I}; and wherein the multilayer article is rigid at temperatures less than T_{g}^{I} and is flexible at temperatures greater than T_{g}^{I}. In some embodiments, the method further includes heating the laminate plate to a temperature greater than T_{g}^{I} to deform the laminate plate, wherein the laminate plate is deformable in at least one direction; and securing the deformed laminate plate to the plurality of bone fragments. In some embodiments, the method includes repeating the heating step a plurality of times.

In another embodiment, the method further includes providing a thermally releasing spring element including a coiled multilayer article of alternating layers of a layer of thermoplastic material and a base layer, the thermoplastic layer having a first surface and a second surface opposite the first surface and the base layer have a first base surface and a second base surface opposite the first base surface, wherein the first base surface of the base layer is adjacent to and permanently bonded to the second surface of the thermoplastic layer, wherein the thermoplastic layer has a first glass transition temperature, T_{g}^{I}, and wherein the multilayer article is rigid at temperatures less than T_{g}^{I} and is flexible at temperatures greater than T_{g}^{I}. In some embodiments, a method includes heating the thermally releasing spring element to a temperature greater than T_{g}^{I}; and inserting the thermally releasing spring element into holes within the deformed laminate plate to secure the deformed laminate plate to the plurality of bone fragments. In some embodiments, a method includes inserting the thermally releasing spring element into a cavity; and heating the thermally releasing spring element to a temperature greater than T_{g}^{I}, wherein heating the thermally releasing spring element causes the multilayer to at least partially uncoil in the cavity.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention can be embodied in different forms and thus should not be construed as being limited to the embodiments set forth herein.
FIG. 1 shows a cross-sectional view of a multilayer article according to one embodiment of the invention;
FIG. 2 shows a cross-sectional view of a multilayer article according to another embodiment of the invention;
FIG. 3 shows a cross-sectional view of multilayer article according to yet another embodiment of the invention;
FIG. 4A shows a plan view of an example bone plate in accordance with an embodiment of the present invention;
FIG. 4B shows a perspective view of an example bone plate in accordance with an embodiment of the present invention positioned on a human cranium;
FIG. 5 shows a cross-sectional view of an example thermally releasing spring element in accordance with an embodiment of the present invention; and
FIGS. 6A and 6B show an example internal distractor incorporating a thermally releasing spring element in accordance with an embodiment of the present invention.

### DETAILED DESCRIPTION OF THE INVENTION

The present subject matter will now be described more fully hereinafter with reference to the accompanying Figures and Examples, in which representative embodiments are shown. The present subject matter can, however, be embodied in different forms and should not be construed as limited to the embodiments set forth herein. Rather, these embodiments are provided to describe and enable one of skill in the art.

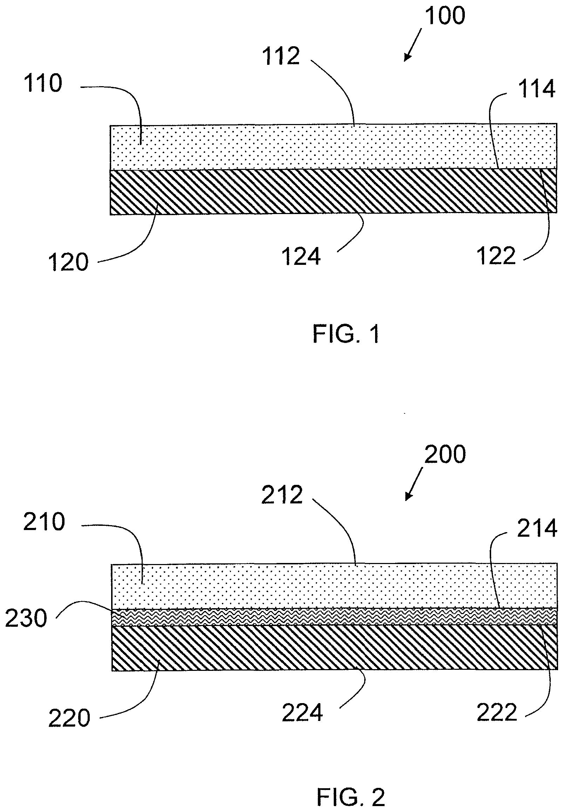

A multilayer article according to one exemplary embodiment of the invention is broadly illustrated in FIG. 1. Referring to FIG. 1, multilayer article 100 includes first thermoplastic layer 110 having first surface 112 and second surface 114 opposite first surface 112. Multilayer article 100 further includes base layer 120 having first surface 122 and second surface 124 opposite first surface 122. In the embodiment shown, first surface 122 of base layer 120 is adjacent to and abuts second surface 114 of thermoplastic layer 110. In certain embodiments, first surface 122 of base layer 120 is affixed to second surface 114 of thermoplastic layer 110. In certain embodiments, first surface 122 is permenantly adhered to second surface 114, for example, by chemical bonds between first surface 122 and second surface 114.

First surface 122 and second surface 114 may be substantially isometric in at least one dimension. In some embodiments, first surface 122 and second surface 114 have substantially the same length. In some embodiments, first surface 122 and second surface 114 have substantially the same width. In some embodiments, first surface 122 and second surface 114 are substantially congruent. In some embodiments, first surface 122 and second surface 114 have substantially equal areas. While thermoplastic layer 110 and base layer 120 are shown in FIG. 1 as having substantially equal thicknesses, it should be understood that thermoplastic layer 110 and base layer 120 may have different thicknesses according to other embodiments of the invention. For example, thermoplastic layer 110 may have a thickness greater than the thickness of base layer 120 in one embodiment. In another embodiment, thermoplastic layer 110 may have a thickness less than the thickness of base layer 120. For purposes of this application, "substantially" means plus or minus 10% of the referenced dimension.

In some embodiments, thermoplastic layer 110 may have a thickness ranging from: 1 mm to 0.01 mm; 1 mm to 0.1 mm; 1 mm to 0.5 mm; and 0.5 mm to 0.01 mm. In other embodiments, thermoplastic layer 110 may have a thickness of: less than 1 mm; less than 0.5 mm; or less than 0.1 mm. In some embodiments, the thermoplastic layer 110 may have a minimum thickness of about 0.01 mm. In some embodiment, base layer 120 may have thickness ranging from: 0.5 mm to 0.01 mm; 0.5 mm to 0.1 mm; 0.5 mm to 0.3 mm; and 0.3 mm to 0.01 mm. In another embodiment, base layer 120 may have thickness of: less than 0.5 mm; less than 0.3 mm; or less than 0.1 mm. In some embodiments, the base layer 120 may have a minimum thickness of about 0.01 mm. In certain embodiments, the thickness of thermoplastic layer 110 is chosen so it retains its shape during deformation and the thickness of base layer 120 is chosen so it is bendable.

FIG. 2 shows a multilayer article according to another exemplary embodiment of the invention. Referring to FIG. 2, multilayer article 200 includes first thermoplastic layer 210 having opposing first and second surfaces 212 and 214, and first base layer 220 having opposing first and second surfaces 222 and 224. Thermoplastic layer 210 is affixed to base layer 220 by at least one interlayer 230 positioned between thermoplastic layer 210 and base layer 220. In some embodiments, interlayer 230 may be permanently bonded to both first surface 222 of base layer 220 and second surface 214 of thermoplastic layer 210. For example, in one embodiment, interlayer 230 is chemically bonded to first surface 222 and/or surface 214. In one embodiment, interlayer 230 is covalently bonded to first surface 222 and/or surface 214.

As with first surface 122 and second surface 114, of the embodiment shown in FIG. 1, and first surface 222 of base layer 220 and second surface 214 of thermoplastic layer 210, of the embodiment shown in FIG. 2, may be substantially congruent, have the same surface area, and/or be substantially isometric in at least one dimension. In one embodiment, first surface 222 and second surface 214 have substantially the same length and/or width. In some embodiments, the thickness of thermoplastic layer 210 may be the same as or different than the thickness of base layer 220. In further embodiments, interlayer 230 may have thickness that is less than the thickness of thermoplastic layer 210 or the thickness of base layer 220. Thermoplastic layer 210 and base layer 220 may have any of the thicknesses as described above for thermplastic layer 110 and base layer 120.

A multilayer article according to other embodiments of the invention can have a plurality of thermoplastic layers and/or base layers. In some preferred embodiments, the thermoplastic layers and base layers are configured in alternating arrangement. In one embodiment, for example, a multilayer article includes a base layer positioned between and attached to two distinct thermoplastic layers. In another embodiment, a multilayer article includes a thermoplastic layer positioned between and attached to two separate base layers. In some embodiments, a multilayer article includes at least two thermoplastic layers. In certain embodiments, a multilayer article includes multiple thermoplastic layers ranging from: 2 to 10 layers; 2 to 50 layers; 5 to 50 layers; 10 to 50 layers; 20 to 50 layers; 30 to 50 layers; and 40 to 50 layers. In certain embodiments, a multilayer article includes: up to 10 double layer units; up to 20 double layer units; up to 30 double layer units; up to 40 double layer units; and up to 50 double layer units, wherein a double layer unit refers to a thermoplastic layer coupled to a base layer, for example, as shown in FIGS. 1 and 2.

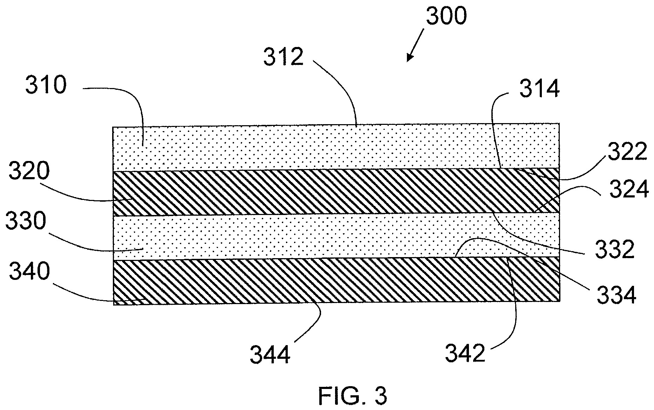

FIG. 3 shows an exemplary multilayer article having a plurality of thermoplastic layers and base layers in accordance with an embodiment of the present invention. Referring to FIG. 3, multilayer article 300 includes first thermoplastic layer 310 having opposing first and second surfaces 312 and 314, first base layer 320 having opposing first and second surfaces 322 and 324, second thermoplastic layer 330 having opposing first and second surfaces 332 and 334, and second base layer 340 having opposing first and second surfaces 342 and 344. First base layer 320 is positioned between first and second thermoplastic layers 310 and 330, and second thermoplastic layer 330 is positioned between first and second base layers 320 and 340. As shown in this embodiment, first surface 322 of first base layer 320 is affixed to second surface 314 of first thermoplastic layer while second surface 324 of first base layer 320 is affixed to first surface 332 of second thermoplastic layer 330. Moreover, second surface 334 of second thermoplastic layer is affixed to first surface 342 of second base layer 340.

The thermoplastic layers of the multilayer articles according to certain preferred embodiments of the present invention are composed of biocompatible polymer materials that become deformable when heated above their glass transition temperatures (Tg). In some embodiments, the biocompatible polymer materials are selected to be substantially rigid at temperatures below their Tg. In some embodiments, the material for the thermoplastic layers is selected to have a Tg in the range of about 35 °C to about 90 °C. In some embodiments, the material for the thermoplastic layers is selected to have a Tg in the range of about 35 °C to about 80 °C. In some embodiments, the material for the thermoplastic layers is selected to have a Tg in the range of about 35 °C to about 70 °C. In some embodiments, the material for the thermoplastic layers is selected to have a Tg above average human body temperature. In some embodiments, the material for the thermoplastic layers is selected to have a Tg above 37 °C.

In some embodiments, the material for the thermoplastic layer is selected so that it is elastic at temperatures greater than its Tg. In some embodiments, the material for the thermoplastic layer is selected to have a melting point below the melting point of the base layer material. In some embodiments, the material for the thermoplastic layer is selected to have a melting point less than 300 °C. In some embodiments, the material for the thermoplastic layer is selected to have a melting point less than 200 °C. In some embodiments, the material for the thermoplastic layer is selected to have a melting point greater than Tg + 20 °C. In some embodiments, the material for the thermoplastic layer is selected to have a melting point greater than Tg + 30 °C. In some embodiments, the material for the thermoplastic layer is selected to have a melting point greater than Tg + 40 °C. In some embodiments, the material for the thermoplastic layer is selected to have a melting point greater than Tg + 50 °C.

In some embodiments, the material for the thermoplastic layer is selected from non-resorbable polymers. In some embodiments, the thermoplastic layer may be made from a material independently selected from: polyethylene terephthalate, polycaproamides, poly(alpha-hydroxy esters), polycaprolactones, polytrimethylene carbonates, polyglycolide-co-trimethylene carbonates, poly(PBA-carbonates), poly(beta-hydroxybutyrate), polyhydroxybutyrate-hydroxyvaleric acids, and combinations thereof.

Certain materials may be unsuitable for the thermoplastic layer according to certain embodiments including resorbable polymers that undergo biodegradation (through the action of water and/or enzymes to be chemically degraded) over a period of time. Examples of resorbable polymers include polylactic acid and its variants, water soluble polymers such as polyvinyl alcohols. Other examples of materials unsuitable for the thermoplastic layer according to certain embodiments include polylactides, poly(alpha-hydroxy esters), poly-L-lactide, poly-DL-lactide, poly-L-lactide-co-DL-lactide, polyglycolic acids, polyglycolide, polylactic-co-glycolic acids, polyglycolide-co-lactide, polyglycolide-co-DL-lactide, and polyglycolide-co-L-lactide.

In certain embodiments wherein a multilayer article includes more than one thermoplastic layer, for example as shown in FIG. 3, two or more of the thermoplastic layers may be made of the same or different materials. In other embodiments, each of the thermoplastic layers in a multilayer article is made of the same material.

The base layers of the multilayer articles according to certain preferred embodiments of the present invention are composed of flexible and/or deformable sheets. In some embodiments, the base layers include flexible and/or deformable foils. In some embodiments, the material for the base layer is selected to exhibit flexural rigidity wherein Young's Modulus, E, is greater than 1 GPa. In some embodiments, the material for the base layer is selected to have a tensile strength greater than 20 MPa. In some embodiments, the material for the base layer is selected to have an elongation to failure value greater than 10 %.

In some embodiments, the base layer is made from a biocompatible metallic material. In some embodiments, the base layer is made from a material substantially resistant to corrosion when implanted in a patient. In some embodiments, the base layer is in the form of a metallic foil. In some embodiments, the base layer may be made from titanium, titanium alloys, cobalt-chrome alloys, cobalt-chrome-molybdenum alloys, molybdenum alloys, tantalum, nitinol, GUMMETAL®, stainless steel, or spring steel such as tempered spring steel. Certain materials may be unsuitable for the base layer according to certain embodiments including biodegrading alloys such as Mg, Fe, Ca, alloys exhibiting stress-corrosion cracking or embrittlement or corroding metals such as Mg or Fe.

In other embodiments, which are not part of the invention, the base layer is made from a high-strength biocompatible polymer material. In such embodiments, the high-strength biocompatible polymer material may have a
tensile strength of at least 90 MPa. In some embodiments, the base layer is made from a polymer resistant to corrosion or degradation when implanted in a patient. In embodiments wherein the base layer is made from a polymer material, preferably the material of the base layer has a glass transition temperature at least 50 °C higher than the Tg of the material selected for the thermoplastic layer. In some embodiments, the base layer may be made from a material selected from the group consisting of: polycarbonate, polyaryl ether ketone, polyether ether ketone, polyether ketone ketone, fiber reinforced polyether ether ketone and fiber reinforced polyether ketone ketone.

In some embodiments, for example as shown in FIG. 2, a multilayer article according to the present invention includes an interlayer between a thermoplastic layer and a base layer. In some embodiments, the interlayer aids in joining the thermoplastic layer and the base layer. In some embodiments, the interlayer contains different surface characteristics than the base layer, for example, having higher wettability and/or adhesion to the thermoplastic layer. In some embodiments, the interlayer demonstrates higher hydrophilicity than the base layer. In some embodiments, the interlayer may be subjected to surface activation to further enhance bonding with the thermoplastic layer. In preferred embodiments, the material of the thermoplastic layer demonstrates a contact angle of about 5° or less at the interface with the interlayer.

In some embodiments, the interlayer includes a diamond-like carbon (DLC) coating which, for example, is applied to a surface of a base layer to which a thermoplastic layer is to be affixed. In some embodiments, the DLC coating can be subjected to surface activation. In some embodiments, the DLC coating is subjected to oxygen plasma treatment.

In some embodiments, the interlayer includes materials with surface groups which may be activated by oxygen plasma. In such embodiments, materials include polymeric materials resulting from the deposition of organometallic alkoxide materials including titanium tetraethoxide, titanium tetra-n-propoxide, titanium tetraisopropoxide, titanium tetra-n-butoxide and titanium tetra-t-butoxide. In other such embodiments, materials include amorphous silicon carbide layers resulting from deposition of tetramethylsilane, hexamethyldisilazane and hexamethyldisiloxane. In some embodiments, the interlayer includes chemical primers, for example, silanes.

Multilayer articles of certain preferred embodiments of the invention are designed to be substantially rigid at a first state and substantially deformable in a second state. In particular, the multilayer articles according to some embodiments become deformable when heated to a temperature above the Tg of their thermoplastic layers. At temperatures below Tg, in these embodiments, the thermoplastic layers remain substantially rigid thereby preventing the base layer(s) affixed thereto from deforming. When the temperature of the thermoplastic layers is higher than Tg, the thermoplastic layers become deformable, which also permits deformation of the base layer(s) affixed thereto. Preferably the transition from the substantially rigid state to the substantially deformable state of the multilayer articles is reversible, for example, such that the multilayer article becomes substantially rigid again once cooled to a temperature below Tg and retaining changes in shape imparted onto multilayer article in the deformable state. For the purpose of this application, substantially deformable means bendable by a force less than 200 N. For the purpose of this application, substantially rigid means not bendable by a force of at least 210 N.

The ability of the multilayer article to reversibly transition from a substantially rigid state to a substantially deformable state is particularly advantageous, in some embodiments, for use in producing customizable surgical implants. In some embodiments, the multilayer article of the present invention may be used to construct bone plates. The bone plates may have any general shape known in the art suitable for bone fixation, osteosynthesis, compression and/or bone fusion. Exemplary bone plates include one-dimensional bone plates. In some embodiments, the bone plates may have a generally linear or longitudinal configuration, for example as shown in U.S. Patent Application Publication No. US 2003/0004515 A1. Other exemplary bone plates that may be constructed from the multilayer articles of the present invention can be found in U.S. Patent Application Publication No. US 2008/0009872 A1. In some embodiments, the bone plates constructed from the multilayer articles of the present invention are non-resorbable.

A bone plate constructed from a multilayer article according to certain embodiments of the present invention can be substantially rigid at body temperature (e.g., about 37 °C) but be deformed when heated to a temperature above Tg. In some embodiments, the bone plate may be heated to a temperature above Tg through exposure to a hot water bath, hot plate, irradiation, heat lamp, or other heat source known in the art. Accordingly, after heating to a temperature above Tg, the bone plate can be shaped or contoured to match a patient's specific anatomy. After cooling the bone plate to a temperature below Tg, the bone plate returns to a substantially rigid state while retaining the shape or contour to match the patient's anatomy.

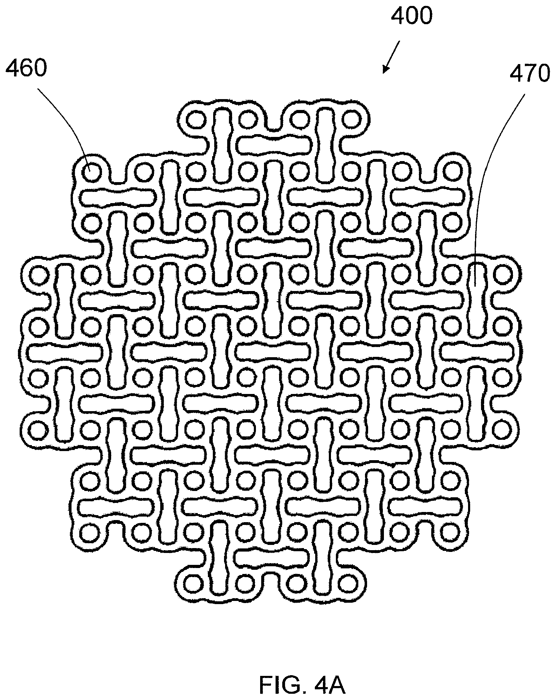

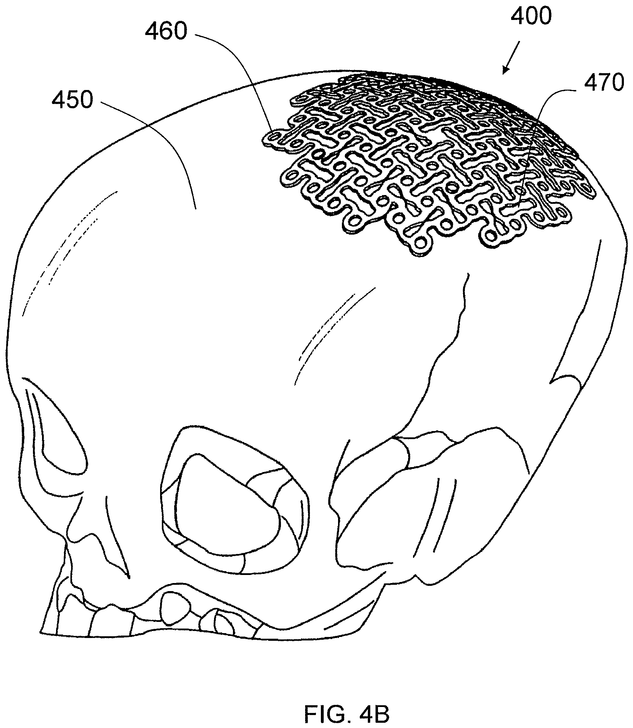

FIGS. 4A and 4B show an example bone plate 400 in accordance with an embodiment of the present invention. Bone plate 400 includes one or more biocompatible thermoplastic layers and one or more biocompatible base layers in accordance with embodiments described herein. In some embodiments, bone plate 400 defines one or more holes 460. In some embodiments, holes 460 are configured to receive a fastener therein, such as a bone screw, in order to secure bone plate 400 onto a surface. Holes 460 may or may not be countersunk. In the embodiment shown, holes 460 are substantially circular. In other embodiments, holes 460 may have any other shape or configuration known in the art. In some embodiments, bone plate 400 further defines one or more elongated slots 470 as shown. In some embodiments, holes 460 and/or elongated slots 470 extend through the entire thickness of bone plate 400 and may be formed by drilling through the layers.

According to some embodiments, bone plate 400 is substantially rigid at temperatures below the Tg value of the thermoplastic layers that it is constructed from. Upon heating bone plate 400 to a temperature above Tg, bone plate 400 may be deformed into any desired shape. Referring to FIG. 4B, for example, bone plate 400 may be contoured to conform to a patient's cranium 450 in one embodiment. In other embodiments, bone plate 400 may be deformed to fit upon other bones or anatomical structures of the patient.

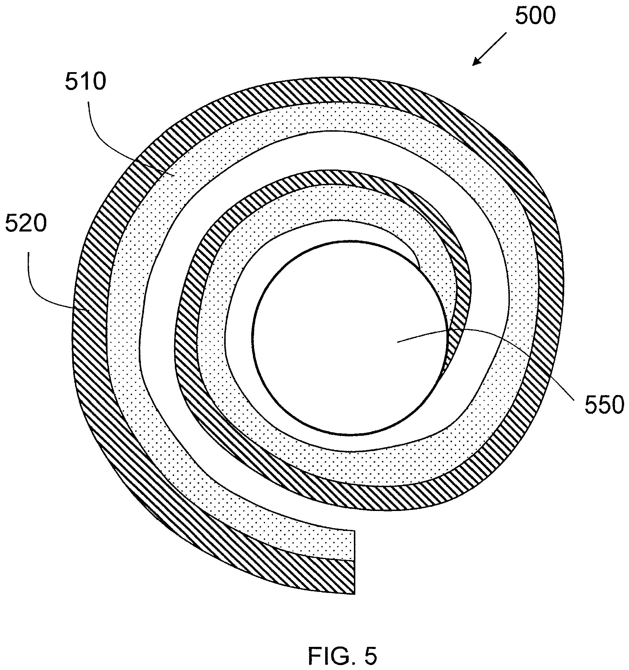

In another embodiment, as shown in FIG. 5, a multilayer article according to the present invention can be configured as a thermally releasing spring element 500. In this embodiment, the thermally releasing spring element 500 includes at least a first thermoplastic layer 510 and at least a first base layer 520 made from an elastic material. In one embodiment, base layer 520 is made from spring steel such as tempered spring steel. In another embodiment, base layer 520 is made from Nitinol or GUMMETAL®. Thermoplastic layer 510 may be made from any of the thermoplastic materials described herein. While spring element 500 is shown having a single base layer 520 and a single thermoplastic layer 510, it should be understood that spring element 500 may have a plurality of base layers and a plurality of thermoplastic layers as described above, for example, in alternating arrangement.

When heated to a temperature above Tg for the thermoplastic layer 510, the thermally releasing spring element 500 becomes deformable and can be tensioned, for example, by coiling or rolling. When subsequently cooled to a temperature below Tg in the coiled or rolled configuration, the thermoplastic layer 510 because substantially rigid thereby retaining the coiled or rolled configuration of the thermally releasing spring element 500 with the elastic base layer 520 remaining under tension. If heated again to a temperature above Tg while unconstrained, the thermally releasing spring element 500 will uncoil as the elastic base layer 520 returns to its initial untensioned state. In some embodiments, the thermally releasing spring element 500 can include a central rod 550 around which the thermoplastic layer 510 and base layer 520 may be coiled. In some embodiments, spring element 500 expands radially from central rod 550 as it uncoils.

In some embodiments, the thermally releasing spring element 500 can be used for fixation. Such thermally releasing spring elements may expand into existing or pre-drilled cavities. In such embodiments, the thermally releasing spring element 500 may lock an anchor in place without other mechanical devices. In some embodiments, the thermally releasing spring elements may take the form of an unthreaded screw. In other embodiments, the thermally releasing spring elements may take the form of a thread screw for improved pull-out resistance. In one such embodiment, a pin may have a thermally releasing spring wound around it which is fixed by the thermoplastic layer until released.

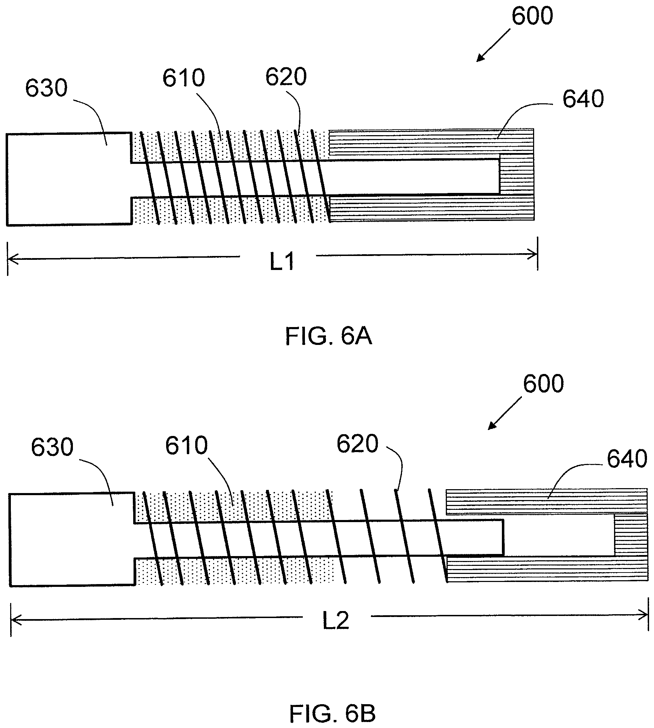

FIGS. 6A and 6B illustrate another embodiment of a thermally releasing spring used in an internal distractor 600 wherein a spring 620 is compressed and immobilized within a thermoplastic material 610 in a first configuration shown in FIG. 6A. In one such embodiment, spring 620 is disposed between and fixed on both ends to implant components 630 and 640. In this first configuration, internal distractor 600 can have a first length L1.

In some embodiments, spring 620 is made from an elastic material. In one embodiment, spring 620 is made from spring steel such as tempered spring steel. In other embodiments, spring 620 is made from nitinol or GUMMETAL®. In some embodiments, thermoplastic material 610 at least partially surrounds spring 620 and may be made from any of the thermoplastic materials described herein. In some embodiments, when thermoplastic material 610 is heated to a temperature at or above its Tg, spring 620 is allowed to expand (e.g., in an axial direction) to a second configuration shown in FIG. 6B. In some embodiments, internal distractor 600 in the second configuration has a second length L2, which is greater than first length L1.

The spring 620 applies a defined force to the components 630 and 640 by virtue of the load previously held back by the thermoplastic when internal distractor 600 transitions from the first configuration to the second configuration. According to some embodiments, internal distractor 600 can be used in a medical implant. In some embodiments, internal distractor 600 can be used to separate or maintain a gap between bones or bone fragments to which components 630 and 640 are affixed. In one such embodiment, internal distractor 600 may be used in the mandible of a patient, for example, for the purposes described in U.S. Patent No. 6,666,869. In one embodiment, components 630 and 640 may be bone plates or anchors affixed to bones separated previously by osteotomy. Following osteotomy, distractor 600 can be used to slowly widen the gap between the separated bones by distraction as it expands, the gap being filled with new bone material for correction of deformities. In some embodiments, where internal distractor 600 is implanted, thermoplastic material 610 can be heated above Tg through the skin, for example, by infrared radiation or inductive heating. In some embodiments, inductive heating can be achieved by introducing a current into spring 620.

A method for making a multilayer article according to an embodiment of the present invention includes one or more of the following steps:
a. providing a base layer;
b. optionally treating a surface of the base layer;
c. superimposing a thermoplastic layer onto the base layer;
d. melting the thermoplastic layer onto the base layer to form a laminate;
e. cooling the laminate; and
f. shaping the laminate.

In some embodiments, treating the surface of the base layer includes modifying the surface of the base layer to improve bonding with the thermoplastic layer. In some embodiments, treating the surface of the base layer includes modifying the surface of the base layer to improve wettability of the base layer. In some embodiments, treating the surface of the base layer includes modifying the surface of the base layer to increase hydrophilicity of the base layer. In some embodiments, treating the surface of the base layer includes cleaning the surface of the base layer, for example, using an organic solvent. In some embodiments, the surface of the base layer is treated to remove oxides, for example, via treatment with a base, sputtering by noble gas, and/or hydrogen ion beam exposure.

In some embodiments, treating the surface of the base layer includes surface activation, for example, by plasma treatment. In some embodiments, plasma treatment creates reactive groups on the surface of the base layer which are capable of forming chemical bonds with the thermoplastic layer or an interlayer. In some embodiments, which are not part of the invention, the base layer is made from a high-strength biocompatible polymer material as discussed previously herein. In other embodiments according to the invention, the base layer is made from a biocompatible metallic material as discussed previously herein. In such embodiments, treating the surface of the base layer includes adding an interlayer that can also be subjected to subsequent surface activation. In some such embodiments, treating the surface of the base layer includes providing an interlayer as discussed previously herein. Such interlayers may be deposited onto the base layer via chemical vapor deposition, plasma assisted chemical vapor deposition or physical vapor deposition. In some embodiments, the interlayer material includes a DLC coating which is subjected to oxygen plasma treatment.

In such embodiments, the plasma treatment leads to formation of reactive groups, such as -OH groups, on the hydrocarbon material, which can chemically connect to neighboring groups located on the thermoplastic layer. In some embodiments, the base layer is treated with vacuum oxygen plasma treatment. Exemplary conditions include radio frequency plasma at a pressure of 1·10⁻² mbar oxygen and plasma power 20 Watts for area of 100 cm². The plasma treatment leads to formation of reactive groups (-OH) on the hydrocarbon material, which can chemically connect to neighboring groups, e.g., a molten thermoplastic.

In some embodiments, surface activation is achieved through exposure of the base layer to UV radiation. Such activation may break bonds within the base layer material and subsequent desorption, leading to generation of a reactive surface. In some embodiments, the UV radiation used to treat the base layer is selected to have a wavelength of about 10 nm to about 260 nm.

In other embodiments, the surface of the base layer may be activated by removing an oxide layer from the surface by treating with basic compounds, sputtering the layer with a noble gas or hydrogen ion beam.

In some embodiments, a thermoplastic layer is superimposed onto the base layer, for example, after the surface of the base layer has been treated or activated. In some embodiments, a method for making a multilayer article includes preparing a stack of alternating base layers and thermoplastic layers. In some embodiments, the stack is formed by reactive injection molding of suitable monomers. In some embodiments, the thermoplastic layer is melted onto the base layer. In some embodiments, melting the thermoplastic layer onto the base layer includes heating the layers under pressure for example at temperatures of at least about 200 °C. In some embodiments, the layers are heated under pressure using a heated press. In some embodiments, the layers are heated under pressure using one or more heated rollers. In other embodiments, the layers are heated by microwave heating, induction heating, IR irradiation or DC current.

In some embodiments, shaping the laminate includes cutting the laminate into a desired final shape. In some embodiments, the laminate is shaped using water jet cutting. In some embodiments, the laminate may be milled or drilled. In some embodiments, the laminate may be laser cut. In some embodiments, the laminate may be shaped by bending, deforming, molding, or rolling the laminate into a desired configuration. In some embodiments, shaping the laminate includes rolling the laminate to form a coil spring shape. In some embodiments, the laminate is shaped at a temperature at or above the glass transition temperature T_{g} of the thermoplastic layer. In some embodiments, the laminate is shaped at a temperature between T_{g} and the melting temperature Tₘ of the thermoplastic layer.

### EXAMPLE 1

An osteosynthesis plate which becomes deformable above the glass transition temperature of polyethylene terephthalate (PET) was fabricated by the following method:
1. The surface of a 0.05 mm thick titanium (Ti) foil was treated by coating with a DLC layer and subsequent surface activation with oxygen plasma. First the titanium foil was ultrasonically cleaned in an organic solvent and then dried. The cleaned and dried titanium foil was placed in a vacuum system, evacuated to a base pressure of approximately 10⁻⁷ mbar. The titanium foil was then cleaned using an argon bombardment, carried out at approximately 2·10⁻² mbar. A DLC layer was then deposited onto the surface of the titanium foil. The DLC layer can have a thickness as desired, for instance less than approximately 10 µm. The DLC layer was deposited onto the substrate by deposition from acetylene. The DLC layer was then activated by RF discharge; pressure 1·10⁻² mbar oxygen, plasma power 20 Watts for area of 100 cm², treatment time 5 minutes. The DLC coated Ti foil was removed from the vacuum chamber.
2. A stack of 10 alternating layers of the DLC treated Ti foil and 0.1 mm thickness polyethylene terephthalate (PET) foil of size 10 x 1 cm was prepared.
3. A pressure of 1 kg/cm² was applied to the stack in a heatable press.
4. The stack was heated to 280 °C for 1 minute to melt the PET and join the layers.
5. The stack was cooled to room temperature and removed from the press.
6. The resulting plate was shaped using water jet cutting.

### EXAMPLE 2

A deformable cranial flap implant was produced by the following method:
1. The surface of a 0.01 mm thick polyether ether ketone (PEEK) foil was activated by vacuum oxygen plasma treatment.
2. A stack of 100 iterations of the PEEK foil and 0.05 mm thick PET foil was prepared.
3. A pressure of 10 kg/cm² was applied to the stack in a heatable press.
4. The stack was heated to 280 °C for 3 minutes to melt the PET and join the layers.
5. The stack was cooled to room temperature and removed from the press.
6. The resulting plate was shaped using water jet cutting.

It should be understood that various changes, substitutions, and alterations can be made herein without departing from the scope of the invention as defined by the appended claims. Moreover, the scope of the present application is not intended to be limited to the particular embodiments of the process, machine, manufacture, and composition of matter, means, methods and steps described in the specification. As one of ordinary skill in the art will readily appreciate from the disclosure herein, processes, machines, manufacture, composition of matter, means, methods, or steps, presently existing or later to be developed that perform substantially the same function or achieve substantially the same result as the corresponding embodiments described herein may be utilized according to the present invention.

## Claims

1. A multilayer article (100; 200; 300) comprising:
a thermoplastic layer (110; 210; 310, 330) having a first surface (112; 212; 312, 332) and a second surface (114; 214; 314, 334) opposite the first surface (112; 212; 312, 332); and
a base layer (120; 220; 320, 340) having a first base surface (122; 222; 322, 342) and a second base surface (124; 224; 324, 344) opposite the first base surface (122; 222; 322, 342);
wherein the first base surface (122; 222; 322, 342) of the base layer (120; 220; 320, 340) is permanently bonded to the second surface (114; 214; 314, 334) of the thermoplastic layer (110; 210; 310, 330), wherein the thermoplastic layer (110; 210; 310, 330) comprises a first thermoplastic material having a first glass transition temperature, T_{g}^{I}; and wherein the multilayer article (100; 200; 300) is rigid at temperatures less than T_{g}^{I} and is flexible at temperatures greater than T_{g}^{I},
**characterized in that** the base layer (120; 220; 320, 340) comprises a biocompatible metallic material.

2. The multilayer article of claim 1, further comprising a plurality of thermoplastic layers (310, 330) and plurality of base layers (320, 340) arranged in alternating layers.

3. The multilayer article of any of claims 1 and 2, wherein the thermoplastic layer (110; 210; 310, 330) comprises a material selected from the group consisting of: polyethylene terephthalate, polycaproamides, polycaprolactones, polytrimethylene carbonates, polyglycolide-co-trimethylene carbonates, poly(PBA-carbonates), poly(beta-hydroxybutyrate), polyhydroxybutyrate-hydroxyvaleric acids, and combinations thereof.

4. The multilayer article of any of claims 1 to 3, wherein the biocompatible metallic material comprises a material selected from the group consisting of: titanium, titanium alloys, cobalt-chrome alloys, cobalt-chrome-molybdenum alloys, molybdenum alloys, tantalum, nitinol, GUMMETAL®, stainless steel and spring steel.

5. The multilayer article of any of claims 1 to 4, wherein the thermoplastic layer (210) and the base layer (220) are permanently bonded by a diamond like carbon interlayer (230) positioned between the thermoplastic layer (210) and the base layer (220).

6. The multilayer article of any of claims 1 to 5, having a thickness ranging from 0.1 mm to 3.0 mm.

7. The multilayer article of any of claims 1 to 6, having a thickness ranging from 0.1 mm to 1.5 mm.

8. A bone plate (400) made from the multilayer article (100; 200; 300) of any of claims 1 to 7.

9. A thermally releasing spring element (500; 620) having a coil spring shape made from the multilayer article (100; 200; 300) of any of claims 1 to 7.

10. A method for forming a multilayer article (100; 200; 300) comprising the steps of:
providing a base layer (120; 220; 320, 340) having a first base surface (122; 222; 322, 342) and a second base surface opposite the first base surface (122; 222; 322, 342);
applying a thermoplastic layer (110; 210; 310, 330) to the first base surface (122; 222; 322, 342) of the base layer (120; 220; 320, 340) to form a multilayer article (100; 200; 300), the thermoplastic layer (110; 210; 310, 330) having a melting temperature, Tₘ, and a first glass transition temperature, T_{g}^{I};
applying pressure to the multilayer article (100; 200; 300);
heating the multilayer article (100; 200; 300) to at least 10 degrees C over Tₘ for at least 1 minute to permanently affix the thermoplastic layer to the surface of the base layer (120; 220; 320, 340), and
wherein the multilayer article (100; 200; 300) is rigid at temperatures less than T_{g}^{I} and is flexible at temperatures greater than T_{g}^{I},
**characterized in that** the base layer (120; 220; 320, 340) comprises a biocompatible metallic material.

11. The method of claim 10, further comprising the steps of:
coating the surface (222) of the biocompatible metallic material with diamond like carbon to form a diamond like carbon interlayer (230) positioned between the thermoplastic layer (210) and the base layer (220); and
activating the diamond like carbon layer (230) with oxygen plasma.

12. The method of claim 10 or 11, further comprising the steps of:
cooling the multilayer article (100; 200; 300), and
shaping the multilayer article (100; 200; 300) to form a deformable implant (400).

13. The method of claim 10 or 11, further comprising the step of:
rolling the multilayer article (100; 200; 300) to form a coil spring shape.

## Patentansprüche

1. Mehrschichtartikel (100; 200; 300), umfassend:
eine thermoplastische Schicht (110; 210; 310, 330), die eine erste Oberfläche (112; 212; 312, 332) und eine zweite Oberfläche (114; 214; 314, 334) gegenüber der ersten Oberfläche (112; 212; 312, 332) aufweist; und
eine Basisschicht (120; 220; 320, 340), die eine erste Basisoberfläche (122; 222; 322, 342) und eine zweite Basisoberfläche (124; 224; 324, 344) gegenüber der ersten Basisoberfläche (122; 222; 322, 342) aufweist;
wobei die erste Basisoberfläche (122; 222; 322, 342) der Basisschicht (120; 220; 320, 340) mit der zweiten Oberfläche (114; 214; 314, 334) der thermoplastischen Schicht (110; 210; 310, 330) permanent verbunden ist, wobei die thermoplastische Schicht (110; 210; 310, 330) ein erstes thermoplastisches Material umfasst, das eine erste Glasübergangstemperatur, T_{g}^{I}, aufweist; und wobei der Mehrschichtartikel (100; 200; 300) bei Temperaturen kleiner als T_{g}^{I} fest ist und bei Temperaturen größer als T_{g}^{I} flexibel ist,
**dadurch gekennzeichnet, dass** die Basisschicht (120; 220; 320, 340) ein biokompatibles metallisches Material umfasst.

2. Mehrschichtartikel nach Anspruch 1, des Weiteren umfassend eine Mehrzahl von thermoplastischen Schichten (310, 330) und eine Mehrzahl von Basisschichten (320, 340), die in abwechselnden Schichten angeordnet sind.

3. Mehrschichtartikel nach einem der Ansprüche 1 und 2, wobei die thermoplastische Schicht (110; 210; 310, 330) ein Material umfasst, ausgewählt aus der Gruppe, bestehend aus: Polyethylenterephthalat, Polycaproamide, Polycaprolactone, Polytrimethylencarbonate, Polyglycolid-Co-Trimethylencarbonate, Poly(PBA-Carbonate), Poly(beta-Hydroxybutyrat), Polyhydroxybutyrat-Hydroxyvaleriansäuren und Kombinationen davon.

4. Mehrschichtartikel nach einem der Ansprüche 1 bis 3, wobei das biokompatible metallische Material ein Material umfasst, ausgewählt aus der Gruppe, bestehend aus: Titan, Titanlegierungen, Cobalt-Chrom-Legierungen, Cobalt-Chrom-Molybdän-Legierungen, Molybdän-Legierungen, Tantal, Nitinol, GUMMETAL®, Edelstahl und Federstahl.

5. Mehrschichtartikel nach einem der Ansprüche 1 bis 4, wobei die thermoplastische Schicht (210) und die Basisschicht (220) durch eine amorphe Kohlenstoff-Zwischenschicht (230), die zwischen der thermoplastischen Schicht (210) und der Basisschicht (220) positioniert ist, permanent verbunden sind.

6. Mehrschichtartikel nach einem der Ansprüche 1 bis 5, der eine Dicke im Bereich von 0,1 mm bis 3,0 mm aufweist.

7. Mehrschichtartikel nach einem der Ansprüche 1 bis 6, der eine Dicke im Bereich von 0,1 mm bis 1,5 mm aufweist.

8. Knochenplatte (400), hergestellt aus dem Mehrschichtartikel (100; 200; 300) nach einem der Ansprüche 1 bis 7.

9. Thermisch entspannendes Federelement (500; 620), das eine Spiralfederform aufweist, hergestellt aus dem Mehrschichtartikel (100; 200; 300) nach einem der Ansprüche 1 bis 7.

10. Verfahren zum Bilden eines Mehrschichtartikels (100; 200; 300), umfassend die Schritte:
Bereitstellen einer Basisschicht (120; 220; 320, 340), die eine erste Basisoberfläche (122; 222; 322, 342) und eine zweite Basisoberfläche gegenüber der ersten Basisoberfläche (122; 222; 322, 342) aufweist;
Aufbringen einer thermoplastischen Schicht (110; 210; 310, 330) auf die erste Basisoberfläche (122; 222; 322, 342) der Basisschicht (120; 220; 320, 340), um einen Mehrschichtartikel (100; 200; 300) zu bilden, wobei die thermoplastische Schicht (110; 210; 310, 330) eine Schmelztemperatur, Tₘ, und eine erste Glasübergangstemperatur, T_{g}^{I}, aufweist;
Aufbringen von Druck auf den Mehrschichtartikel (100; 200; 300);
Erwärmen des Mehrschichtartikels (100; 200; 300) auf zumindest 10 Grad C über Tₘ für zumindest 1 Minute, um die thermoplastische Schicht an der Oberfläche der Basisschicht (120; 220; 320, 340) permanent zu fixieren, und
wobei der Mehrschichtartikel (100; 200; 300) bei Temperaturen kleiner als T_{g}^{I} fest ist und bei Temperaturen größer als T_{g}^{I} flexibel ist,
**dadurch gekennzeichnet, dass** die Basisschicht (120; 220; 320, 340) ein biokompatibles metallisches Material umfasst.

11. Verfahren nach Anspruch 10, des Weiteren umfassend die Schritte:
Beschichten der Oberfläche (222) des biokompatiblen metallischen Materials mit amorphem Kohlenstoff, um eine amorphe Kohlenstoff-Zwischenschicht (230) zu bilden, die zwischen der thermoplastischen Schicht (210) und der Basisschicht (220) positioniert ist; und
Aktivieren der amorphen Kohlenstoffschicht (230) mit Sauerstoffplasma.

12. Verfahren nach Anspruch 10 oder 11, des Weiteren umfassend die Schritte:
Abkühlen des Mehrschichtartikels (100; 200; 300) und
Formen des Mehrschichtartikels (100; 200; 300), um ein verformbares Implantat (400) zu bilden.

13. Verfahren nach Anspruch 10 oder 11, des Weiteren umfassend den Schritt:
Aufrollen des Mehrschichtartikels (100; 200; 300), um eine Spiralfederform zu bilden.

## Revendications

1. Article multicouche (100; 200; 300), comprenant :
une couche thermoplastique (110; 210; 310; 330) présentant une première surface (112; 212; 312; 332) et une seconde surface (114; 214; 314; 334) face à la première surface (112; 212; 312; 332), et
une couche de base (120; 220, 320, 340) présentant une première surface de base (122; 222; 322; 342), et une seconde surface de base (124; 224, 324, 344) face à la première surface de base (122; 222; 322; 342) ;
dans lequel la première surface de base (122; 222; 322; 342) de la couche de base (120; 220, 320, 340) est liée de manière permanente à la seconde surface (114; 214; 314; 334) de la couche thermoplastique (110; 210; 310; 330) ; dans lequel la couche thermoplastique (110; 210; 310; 330) comprend un premier matériau thermoplastique présentant une première température de transition vitreuse T¹_{g}, et dans lequel l'article multicouche (100; 200; 300) est rigide à des températures inférieures à T¹_{g}, et flexible à des températures supérieures à T¹_{g},
**caractérisé en ce que** la couche de base (120; 220, 320, 340) comprend un matériau métallique biocompatible.

2. Article multicouche selon la revendication 1, comprenant en outre une pluralité de couches thermoplastiques (310, 330) et une pluralité de couches de base (320, 340) agencées en couches alternées.

3. Article multicouche selon la revendication 1 ou 2, dans lequel la couche thermoplastique (110; 210; 310; 330) comprend un matériau sélectionné parmi : du téréphtalate de polyéthylène, des polycaproamides, des polycaprolactones, des carbonates de polytriméthylène, des carbonates de polyglycolide-cotriméthylene, des poly(PBA-carbonates), des poly(beta-hydroxybutyrate), des acides polyhydroxybutyrate-hydroxyvalériques, et des combinaisons de ceux-ci.

4. Article multicouche selon l'une quelconque des revendications 1 à 3, dans lequel le matériau métallique biocompatible comprend un matériau sélectionné parmi : du titane, des alliages de titane, des alliages cobalt-chrome, des alliages cobalt-chrome-molybdène, des alliages de molybdène, du tantale, du nitinol, du GUMMETAL®, de l'acier inoxydable, et de l'acier à ressorts.

5. Article multicouche selon l'une quelconque des revendications 1 à 4, dans lequel la couche thermoplastique (210) et la couche de base (220) sont liées de manière permanente par une couche intermédiaire de carbone de type diamant (230) positionnée entre la couche thermoplastique (210) et la couche de base (220).

6. Article multicouche selon l'une quelconque des revendications 1 à 5, présentant une épaisseur allant de 0,1 mm à 3,0 mm.

7. Article multicouche selon l'une quelconque des revendications 1 à 6, présentant une épaisseur allant de 0,1 mm à 1,5 mm.

8. Lame osseuse (400) fabriquée à partir de l'article multicouche (100; 200; 300) selon l'une quelconque des revendications 1 à 7.

9. Elément formant ressort à déclenchement thermique (500; 620) présentant une forme de ressort hélicoïdal fabriqué à partir de l'article multicouche (100; 200; 300) selon l'une quelconque des revendications 1 à 7.

10. Procédé destiné à former un article multicouche (100; 200; 300), comprenant les étapes consistant à :
fournir une couche de base (120; 220, 320, 340) présentant une première surface de base (122; 222; 322; 342), et une seconde surface de base face à la première surface de base (122; 222; 322; 342);
appliquer une couche thermoplastique (110; 210; 310; 330) sur la première surface de base (122; 222; 322; 342) de la couche de base (120; 220, 320, 340) afin de former un article multicouche (100; 200, 300), la couche thermoplastique (110; 210; 310; 330) présentant une température de fusion, Tₘ, et une première température de transition vitreuse, à T¹_{g},
appliquer une pression à l'article multicouche (100; 200, 300) ;
chauffer l'article multicouche (100; 200, 300) à au moins 10 °C au-dessus de Tₘ pendant au moins une 1 minute pour fixer de manière permanente la couche thermoplastique sur la surface de la couche de base (120; 220, 320, 340), et
dans lequel l'article multicouche (100; 200; 300) est rigide à des températures inférieures à T¹_{g}, et est flexible à des températures supérieures à T¹_{g},
**caractérisé en ce que** la couche de base (120; 220, 320, 340) comprend un matériau métallique biocompatible.

11. Procédé selon la revendication 10, comprenant en outre les étapes consistant à :
revêtir la surface (222) du matériau métallique biocompatible avec du carbone de type diamant pour former une couche intermédiaire de carbone de type carbone (230) positionnée entre la couche thermoplastique (210) et la couche de base (220), et
activer la couche de carbone de type carbone (230) avec un plasma d'oxygène.

12. Procédé selon la revendication 10 ou 11, comprenant en outre les étapes consistant à :
refroidir l'article multicouche (100; 200; 300), et
façonner l'article multicouche (100; 200; 300) pour former un implant déformable (400).

13. Procédé selon la revendication 10 ou 11, comprenant en outre l'étape consistant à :
rouler l'article multicouche (100; 200; 300) pour donner une forme de ressort hélicoïdal.
